# EUROPÄISCHE PATENTANMELDUNG

(11) **EP 4 484 575 A1**
(43) Veröffentlichungstag der Anmeldung: **01.01.2025**
(21) Anmeldenummer: 23182935.9
(22) Anmeldetag: 30.06.2023
(51) Int. Cl.: C12Q 1/6883

(54) **MARKERSEQUENZEN ZUR DIAGNOSE UND THERAPIESTEUERUNG VON PATIENTEN MIT TESTOSTERON-DEFIZIENZ**

(71) Anmelder: Cerascreen GmbH, 19059 Schwerin (DE)
(72) Erfinder: BLUM, Wilfried, Lüneburg (DE)
(74) Vertreter: Simandi, Claus

(57) **Zusammenfassung**

Die vorliegende Erfindung betrifft ein Verfahren zur Diagnose, Prognose, Risikostratifizierung und Therapiesteuerung von Testosteronmangel bzw. Testosterondefizit bei Patienten und Patientenpopulationen sowie die Verwendung von geeigneten Markersequenzen und ein Test-Kit.

## Beschreibung

Die vorliegende Erfindung betrifft ein Verfahren zur Diagnose, Prognose, Risikostratifizierung und Therapiesteuerung von Testosteronmangel bzw. Testosterondefizit bei Patienten und Patientenpopulationen sowie die Verwendung von geeigneten Markersequenzen und ein Test-Kit.

Testosteron (17β-Hydroxyandrost-4-en-3-on)ist ein männliches Sexualhormon bzw. Androgen, das in den Hoden (Gonaden) produziert wird. Es ist bekannt dafür, männliche körperliche Merkmale zu fördern, wie z.B. Muskelmasse und Körperbehaarung, sowie die Libido zu steigern und die Spermienproduktion zu unterstützen.

Ein Testosteronmangel, auch als Hypogonadismus bezeichnet, bezieht sich auf einen Zustand, wenn die Hoden nicht genügend Testosteron produzieren oder wenn der Körper das produzierte Testosteron alters-, verletzungs- oder krankheitsbedingt nicht richtig nutzen kann. Ebenfalls können genetische Ursachen vorliegen.

Ein Testosteronmangel kann viele Auswirkungen auf den Körper haben, einschließlich einer Verringerung der Muskelmasse und - kraft, einer Erhöhung des Körperfetts, einer Abnahme der Knochendichte, einer Reduzierung der Libido und Erektionsstörungen. Es kann auch zu Müdigkeit, Depressionen, Stimmungsschwankungen und einer verringerten geistigen Schärfe führen.

Die Diagnose eines Testosteronmangels wird durch Bluttests zur Messung des Testosteronspiegels im Körper gestellt. Wenn ein Mangel diagnostiziert wird, kann mittels Hormonersatztherapien behandelt werden, wie z.B. der Verabreichung von Testosteroninjektionen, Gelen oder Pflastern.

Die Epigenetik bezieht sich auf Veränderungen in der Genexpression, die nicht auf Änderungen in der DNA-Sequenz selbst zurückzuführen sind. Stattdessen regulieren epigenetische Mechanismen, wie Chromatinstruktur und DNA-Methylierung, welche Gene aktiv oder inaktiv sind und damit welche Proteine produziert werden.

Chromatin ist die Struktur, in der die DNA im Zellkern verpackt ist, und die Methylierung von DNA ist ein Prozess, bei dem Methylgruppen an die DNA gebunden werden, wodurch die Chromatinstruktur verändert wird. Diese epigenetischen Veränderungen können dauerhaft oder vorübergehend sein und durch verschiedene Faktoren wie Alter, Umwelt und Lebensstil beeinflusst werden.

Epigenetische Veränderungen können wichtige Auswirkungen auf die Gesundheit und die Entwicklung haben. Zum Beispiel kann eine Aberration in der Methylierung von bestimmten Genen das Risiko für bestimmte Krankheiten erhöhen oder sogar den Verlauf der Krankheit beeinflussen. Eine Aberration in der Chromatinstruktur kann auch dazu führen, dass bestimmte Gene nicht mehr exprimiert werden, was wiederum zu einer Anomalie in der Entwicklung führen kann.

Die Epigenetik hat auch das Potenzial, zur Entwicklung von Therapien beizutragen, indem sie ermöglicht, bestimmte Gene zu aktivieren oder zu deaktivieren, um bestimmte Erkrankungen zu behandeln. Die Epigenetik wird auch intensiv erforscht, um mehr über die zugrunde liegenden Mechanismen der Genexpression zu erfahren und das Verständnis von Entwicklungsprozessen und Krankheiten zu vertiefen.

CpG-Sites sind DNA-Regionen, die aus einem Cytosin (C) und einem Guanin (G) bestehen, die durch eine Phosphatbindung miteinander verbunden sind. Diese CpG-Dinukleotide sind in der DNA nicht gleichmäßig verteilt, sondern treten in Gruppen auf, die als CpG-Inseln bezeichnet werden.

CpG-Sites spielen eine wichtige Rolle in der Epigenetik, da sie die primäre Zielregion für DNA-Methylierungsreaktionen sind. Bei der DNA-Methylierung wird eine Methylgruppe an das Kohlenstoffatom des Cytosins gebunden, was zur Bildung von 5-Methylcytosin (5mC) führt. Die Methylierung von CpG-Sites kann dazu führen, dass die Genexpression in der umliegenden DNA-Region unterdrückt wird, was zu einer Verminderung oder gar Abschaltung der Genexpression führt.

Tatsächlich werden CpG-Inseln häufig in Promotor-Regionen von Genen gefunden, die mit der Regulation der Genexpression verbunden sind. Eine Aberration in der Methylierung von CpG-Inseln kann die Expression von Genen beeinflussen und zu verschiedenen Krankheiten oder Zuständen beitragen, wobei eine Methylierung oder Demethylierung der CpG-Sites bzw. CpG-Inseln erfolgt. Die häufigste Form ist die sogenannte Hypermethylierung der CpG-Sites bzw. CpG-Inseln. Weiterhin ist die Hypomethylierung beschrieben.

Die Identifizierung von CpG-Sites bzw. CpG-Inseln hat folglich eine wichtige Bedeutung in der Epigenetik-Forschung, insbesondere in der Identifikation von epigenetischen Markern und deren Zusammenhang mit bestimmten Krankheiten.

Überaschenderweise konnten die Erfinder epigenetische Marker für den Zustand des Testosteronmangels bei einem Patienten identifizieren, insbesondere in Abhängigkeit des Alterns.

Daher ist eine Aufgabe der vorliegenden Erfindung die Therapie, Prophylaxe und Verhinderung des Testosteronmangels mittels medikamentöser Behandlung als auch hinsichtlich klinischer Entscheidungen zu verbessern.

Eine weitere Aufgabe der Erfindung ist ebenfalls die Therapiesteuerung mittels einer Prognose oder Diagnose von Testosteronmangel bzw. Testosterondefizit - Patienten. Insbesondere solche Testosterondefizit - Patienten, die bisher keine medikamentöse Behandlung erfahren bzw. vorzugsweise zur Früherkennung solcher Testosterondefizit - Patienten, insbesondere zur Prophylaxe oder Verhinderung eines Testosteronmangels bzw. Testosterondefizits.

Diese Aufgaben werden durch die technische Lehre der Patentansprüche gelöst.

Die Aufgabe wird erfindungsgemäß dadurch gelöst, dass ein Verfahren zur Diagnose oder Prognose von Patienten mit Testosteronmangel bereitgestellt wird, umfassend Analyse des Methylierungsstatus von mindestens einer CpG-Insel einer Markersequenz (MS) ausgewählt aus MS No. 1 - 23 einer Patientenprobe, wobei mindestens eine Modifikation durch Methylierung und / oder Demethylierung im Vergleich mit der jeweiligen MS No. 1 - 23 indikativ ist.

In einer weiteren bevorzugten Ausführungsform betrifft die Erfindung ein Verfahren zur Diagnose oder Prognose von Patienten mit Testosteronmangel, umfassend Analyse des Methylierungsstatus von mindestens einer CpG-Insel einer Markersequenz ausgewählt aus MS No. 1 - 23, wobei eine Hypermethylierung oder Hypomethylierung der jeweiligen MS No. 1 - 23 vorliegt und indikativ ist.

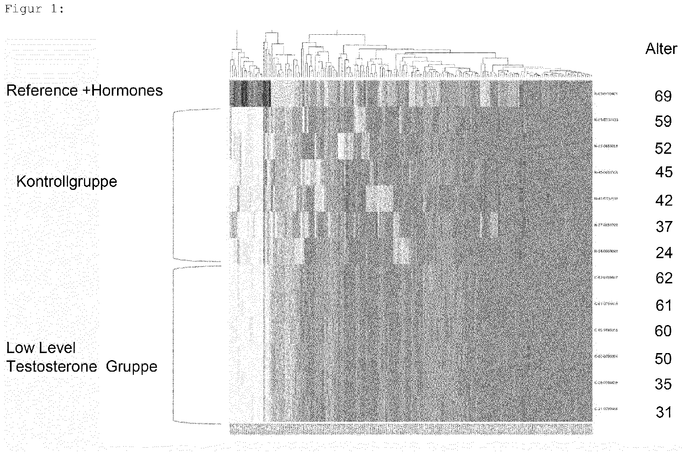

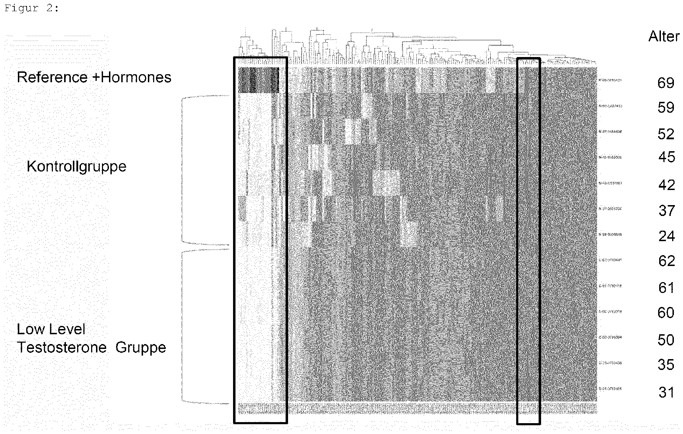

Aus den Ergebnissen der Beispiele zeigt sich die Eignung der erfindungsgemäßen Markersequenzen besonders geeignet für Patienten im Alter von 25 - 60 Jahren, insbesondere 30 - 55 Jahren, siehe Figuren 1 und 2.

In einer weiteren bevorzugten Ausführungsform betrifft die Erfindung daher ein Verfahren zur Diagnose oder Prognose von Patienten mit Testosteronmangel, wobei die Patienten ausgewählt sind aus der Gruppe 30 - 55 Jahre.

Gegenstand der Erfindung ist auch die Verwendung von einer oder mehreren erfindungsgemäßen Markersequenz(en) zur Stratifizierung und Therapiesteuerung von Testosteronmangel bzw. Testosterondefizit - Patienten.

Eine weitere Ausführungsform betrifft die erfindungsgemäße Verwendung dadurch gekennzeichnet, dass 2 oder 3, vorzugsweise 4 oder 5, besonders bevorzugt 6, 7 oder 8 oder mehr verschiedene Markersequenzen, beispielsweise 10 bis 20 verschiedene Markersequenzen verwendet werden. Ein solches Set an Markersequenzen wird Panel genannt.

Gegenstand der Erfindung ist auch ein Verfahren zum Stratifizieren, insbesondere zur Risikostratifizierung, oder zur Therapiesteuerung eines Patienten von Testosteronmangel bzw. Testosterondefizit - Patienten, wobei mindestens eine erfindungsgemäße Markersequenz verwendet wird, um eine Probe des Patienten zu untersuchen.

Ganz besonders geeignet ist das erfindungsgemäße Verfahren zur Frühdiagnose oder Früherkennung von Testosteronmangel, wobei insbesondere keine, also asymptomatisch, oder wenig Symptome (supra) am Patienten zu erkennen sind.

Eine Ausführungsform betrifft ein erfindungsgemäßes Verfahren zur Diagnose von Testosteronmangel, wobei das Stratifizieren oder die Therapiesteuerung Entscheidungen zur Behandlung und Therapie des Patienten, insbesondere Einsatz, Wirkung und / oder Dosierung eines oder mehrerer Arzneimittel, eine therapeutische Maßnahme oder die Überwachung eines Krankheitsverlaufes sowie Therapieverlaufs, Ätiologie oder Klassifizierung einer Erkrankung, ggfs. samt Prognose umfasst.

Daher betrifft die Erfindung die Verwendung von Markersequenzen zur Stratifizierung oder Therapiesteuerung von Testosteronmangel, wobei mindestens eine Markersequenz ausgewählt aus der Gruppe der Markersequenzen MS No 1-23 Teilsequenzen oder Fragmente davon, Homologen der Sequenzen MS No. 1-23 mit mindestens 90 % Homologie zu einem zu untersuchenden Patienten bestimmt wird, wobei mindestens eine Modifikation durch Methylierung und / oder Demethylierung im Vergleich mit der jeweiligen MS No. 1 - 23 indikativ ist.

Eine weitere Ausführungsform der Erfindung betrifft die Verwendung der erfindungsgemäßen Markersequenz(en)zur Stratifizierung oder Therapiesteuerung von Testosteronmangel, wobei die Bestimmung mittels einer in-vitro Diagnose erfolgt.

In einer bevorzugten Ausführungsform erfolgt die Bestimmung der Markersequenzen außerhalb des menschlichen Körpers und die Bestimmung erfolgt in einer ex vivo / in vitro Diagnose.

Die erfindungsgemäßen Markersequenzen konnten mittels differentiellen Screenings von Proben von gesunden Probanden mit Patientenproben mit Testosteronmangel identifiziert werden, siehe Beispiele.

Vorzugsweise erfolgt die Analyse mittels Amplifikation durch ein Polymerase-Enzym, eine PCR oder eine chemische Amplifikationsreaktion oder andere Amplifikationsverfahren, d.h. im Rahmen von MSP, HeavyMethyl, Scorpion, MS-SNUPE, MethylLight, Bisulfit-Sequenzierung, methylspezifische Restriktionsassays und/oder digitale PCR (siehe, zum Beispiel Kristensen und Hansen PCR-Based Methods for Detecting Single-Locus DNA Methylation Biomarkers in Cancer Diagnostics, Prognostics, and Response to Treatment Clinical Chemistry 55:8 1471-1483 (2009)).

Um die Bisulfit-Konvertibilität der CpG-Inseln zu analysieren, kann jedes bekannte Verfahren zur Analyse der DNA-Methylierung verwendet werden. In einer bevorzugten Ausführungsform des Verfahrens gemäß der vorliegenden Erfindung umfasst die Analyse des Methylierungsstatus ein Verfahren ausgewählt aus Methylierungs-spezifischem enzymatischem Verdau, Bisulfit-Sequenzierung, einer Analyse ausgewählt aus Promotermethylierung, CpG-Insel-Methylierung, MSP, HeavyMethyl, MethyLight, Ms-SNuPE oder anderen Methoden, die auf dem Nachweis amplifizierter DNA beruhen. Diese Verfahren sind dem Fachmann gut bekannt und finden sich in der entsprechenden Literatur.

In einer bevorzugten Ausführungsform des Verfahrens gemäß der vorliegenden Erfindung ist das Verfahren für Routineanwendungen, beispielsweise auf einem DNA-Array geeignet. Basierend auf den obigen Informationen und der entsprechenden Literatur kann der Fachmann das obere Verfahren wie oben beschrieben an diese Einstellungen anpassen.

Der Begriff "Testosteronmangel" ist erfindungsgemäß definiert, dass der Patient weniger als 30 pg/ ml oder 40 pg/ ml freies Testosteron im Speichel in der Altersgruppe 30 -65 Jahren aufweist. Die Diagnose und Prognose ist auch dahingehend zu verstehen, dass der Proband in Zukunft von einem Testosteronmangel betroffen sein kann, auch wenn er zum Zeitpunkt der Probenentnahme normale Testosteronwerte aufweist, nämlich 30-140 pg/ ml freies Testosteron im Speichel.

"Diagnose" oder "Prognose" im Sinne dieser Erfindung bedeutet die positive Feststellung oder Vorhersage bzw. Wahrscheinlichkeit des Eintretens von Testosteronmangel bzw. Testosterondefizit mittels der erfindungsgemäßen Markersequenzen sowie die Zuordnung der Patienten zur Indikation Testosteronmangel bzw. Testosterondefizit. Der Begriff der Diagnose umfasst die medizinische Diagnostik und diesbezügliche Untersuchungen, insbesondere die in-vitro Diagnostik und Labordiagnostik.

Ferner betrifft die Erfindung ein Verfahren zum Stratifizieren, insbesondere zur Risikostratifizierung und / oder Therapiesteuerung eines Patienten mit Testosteronmangel bzw. Testosterondefizit, beispielsweise bei einem Patienten mit einem sehr frühen Stadium von Testosteronmangel bzw. Testosterondefizit.

"Stratifizieren oder Therapiesteuerung" im Sinne dieser Erfindung bedeutet, dass das erfindungsgemäße Verfahren Entscheidungen zur Behandlung und Therapie des Patienten erlaubt, sei es Hospitalisierung des Patienten, Einsatz, Wirkung und / oder Dosierung eines oder mehrerer Arzneimittel, eine therapeutische Maßnahme oder die Überwachung eines Krankheitsverlaufes sowie des Therapieverlaufs bzw. Ätiologie oder Klassifizierung von Testosteronmangel bzw. Testosterondefizit.

In einer weiteren Ausführungsform der Erfindung umfasst der Begriff "Stratifizierung" insbesondere die Risikostratifizierung mit der Prognose eines "outcome" eines nachteiligen gesundheitlichen Ereignisses.

Im Rahmen dieser Erfindung wird unter "Patient" ein beliebiger Proband - Mensch oder Säugetier, vorzugsweise mit männlichem Geschlecht - verstanden, mit der Maßgabe, dass der Proband auf Testosteronmangel bzw. Testosterondefizit untersucht wird, insbesondere ein Therapieerfolg oder Therapieverbesserung oder mit der Maßgabe, dass der Proband bzw. das Individuum Medikamente zur Behandlung von Testosteronmangel bzw. Testosterondefizit erhält bzw. erhalten soll, wie beispielsweise mittels einer Hormonersatztherapie.

Die zu untersuchende Sequenzen in einer Probe sind erfindungsgemäß Bestandteil einer Körperflüssigkeit, insbesondere Blut, Vollblut, Blutplasma, Blutserum, Patientenserum, Urin, Cerebrospinalflüssigkeit, Synovialflüssigkeit oder eines Gewebeauszuges des Patienten.

Ganz besonders bevorzugt ist jedoch die Probenentnahme mithilfe eines Wangenabstriches.

Die erfindungsgemäßen Markersequenzen MS No. 1-23 sind Gegenstand der Tabelle 1 und können durch den jeweilig zitierten Datenbankeintrag (Internet: www.http://www.ewascatalog.org/) eindeutig identifiziert werden (siehe cg und Gennamen samt Position).

**Tabelle 1: Markersequenzen (MS) aus Homo sapiens**

| MS No | SEQ ID | cg | Name | Gene Type | Gene |
|---|---|---|---|---|---|
| 1 | 1 | cg 15570860 | TMEM9B antisense RNA 1 | ncRNA | C11orf15 |
| | | | | | Chr11:8986840 |
| 2 | | cg01659459 | protein phosphatase 2 regulatory subunit B'gammaprovided | Protein coding | chr14:102227496 |
| 3 | | cg 16398051 | ADAM metallopeptidase with thrombospondin type 1 motif 17 | Protein coding | chr15:100821467 |
| 4 | 2 | cg26624794 | endoplasmic reticulum-golgi intermediate compartment 1 | Protein coding | chr5:172332685 |
| 5 | | cg24534774 | pre-mRNA processing factor 31 | Protein coding | chr1:101106534 |
| 6 | 3 | cg01600516 | arachidonate 12-lipoxygenase, 12S type | Protein coding | chr17:6904264 |
| 7 | 4 | cg04777551 | major histocompatibility complex, dass II, DQ beta 1 | Protein coding | chr6:32628954 |
| 8 | | cg24643105 | n.a. | n.a. | chr11:113928473 |
| 9 | | cg23476908 | p21 (RAC1) activated kinase 3 | Protein coding | chrX:110365603 |
| 10 | 5 | cg05162750 | T-box transcription factor 2 | Protein coding | chr17:59476506 |
| 11 | | cg27590199 | sarcoma antigen 1 | Protein coding | chrX:134974589 |
| 12 | | cg12342501 | n.a. | Open sea | chr2:8530522 |
| 13 | 6 | cg13072209 | SLC22A23 | Open sea | chr6:3298568 |
| 14 | | cg03165426 | CRHR2 | Open sea | chr7:30726958 |
| 15 | 7 | cg11796481 | NUP93 | Open sea | chr16:56864618 |
| 16 | 8 | cg26495595 | TSGA10 | Open sea | chr2:99696417 |
| 17 | | cg00979438 | na | Open sea | chr16:73199845 |
| 18 | 9 | cg22564046 | PFKP | South shore | chr10:3161236 |
| 19 | 10 | cg19843426 | PARD6G-AS1;PARD6G | Open sea | chr18:77925064 |
| 20 | | cg04531698 | MGST1 | Open sea | chr12:16514921 |
| 21 | 11 | cg22017303 | Gene AGL | Open sea | chr1:100327026 |
| 22 | 12 | cg03029993 | Gene4 KIAA1429 | Open sea | chr8:95538708 |
| 23 | | cg16191297 | Gene TRAPPC9 | Open sea | chr8:140926710 |

In einer weiteren Ausführungsform der Erfindung sind Homologe der erfindungsgemäßen Markersequenzen umfasst. Insbesondere solche Homologen, die eine Identität von 90 %, 91 %, 92 %, 93 %, 94 % oder 95% Identität, insbesondere 96 %, 97 %, 98 %, 99 % oder mehr Identität mit den erfindungsgemäßen Markersequenzen aufweisen und für die erfindungsgemäße Verwendung - den Nachweis von Testosteronmangel bzw. Testosterondefizit geeignet sind (sog. "Homologe", homologe Markersequenzen).

Teilsequenzen oder Fragmente sind solche Sequenzen, die 50 bis 100 Nukleotide, vorzugsweise 100-500, besonders bevorzugt 500 bis 1000 Nukleotide einer der Markersequenzen MS No. 1-23 aufweisen, wie z.B. Oligonukleotide.

Solche Markersequenzen der jeweiligen genomischen DNA zu MS No. 1 - 23 sind in den Sequenzen SEQ ID No. 1-12 ausgewiesen gemäß Tabelle 1 und sind entsprechend zu lesen. Die SEQ ID No. 1-12 sind in den jeweilig zugehörigen MS No. enthalten.

Ein weiterer bevorzugter Aspekt der vorliegenden Erfindung betrifft ein (Test-)Kit zur Durchführung des erfindungsgemäßen Verfahrens, insbesondere basierend auf der Analyse der Bisulfit-Zugänglichkeit von CpG-Inseln der Markersequenzen MS No. 1-23, umfassend Komponenten zur Durchführung des erfindungsgemäßen Verfahrens, insbesondere umfassend optional ein Bisulfitreagenz und Materialien zur Analyse des Methylierungsstatus der CpG-Inseln ausgewählt aus mindestens einer Markersequenz, insbesondere ein Oligomer.

Die Erfindung wird nun in den folgenden Beispielen und unter Bezugnahme auf die beigefügten Figuren und das Sequenzprotokoll näher beschrieben, ohne darauf beschränkt zu sein.

### Beispiele:

Die DNA des Probanden wird mittels Oracollect Probenahmesystem durch einen Abstrich der Epithelzellen der Wangeninnenseite entsprechend den Herstellerangaben genommen.

Die Aufreinigung der DNA zur Analyse erfolgt mittels Qiagen DNA-Aufreinigungskit (QIAamp UCP DNA Micro Kit) entsprechend der Arbeitsanleitung.

Die "Epigenetische Analyse erfolge auf dem Illumina Infinium R MethylationEPIC BeadChip system entsprechend den Herstellerangaben:
Illumina DNA Prep Reference Guide (1000000025416 v10); https://support.illumina.com/documentation.html
Infinium MethylationEPIC v2.0 BeadChip, PDF https://support.illumina.com/documentation.html
Noble AJ, Pearson JF, Boden JM, Horwood LJ, Gemmell NJ, Kennedy MA, Osborne AJ. A validation of Illumina EPIC array system with bisulfite-based amplicon sequencing. PeerJ. 2021 Feb 10;9:e10762. doi: 10.7717/peerj.10762. PMID: 33614276; PMCID: PMC7881719.
Bibikova M, Le J, Barnes B, Saedinia-Melnyk S, Zhou L, Shen R, Gunderson KL. Genome-wide DNA methylation profiling using Infinium® assay. Epigenomics. 2009;1(1):177-200. doi: 10.2217/epi.09.14. -

### Data Analysis

Die Analyse der resultierenden iDat-Dateien erfolgte in R.

Hierbei erfolgt der Vergleich der Methylierungsintensität der genannten Markersequenzen gegen die vorhandenen Standards aus der Epigenetischen Datenbank.

Figuren 1 und 2 zeigen die Heat map von 184 untersuchten GpC Inseln in Abhängigkeit des Alters und Populationsgruppen (Low Level Testosterongruppe und Kontrollgruppe).

## Patentansprüche

1. Verfahren zur Diagnose oder Prognose oder Risikostratifizierung von Patienten mit Testosteronmangel, umfassend Analyse des Methylierungsstatus von mindestens einer CpG-Insel einer Markersequenz ausgewählt aus MS No. 1 - 23 einer Patientenprobe, wobei mindestens eine Modifikation durch Methylierung und / oder Demethylierung im Vergleich mit der jeweiligen MS No. 1 - 23 indikativ ist.

2. Verfahren zur Diagnose oder Prognose oder Risikostratifizierung von Patienten mit Testosteronmangel nach Ansprucch 1, wobei eine Hypermethylierung oder Hypomethylierung der jeweiligen MS No. 1 - 23 vorliegt.

3. Verfahren zur Diagnose oder Prognose oder Risikostratifizierung von Patienten mit Testosteronmangel nach einem der vorstehenden Patentansprüche, wobei die Patienten ausgewählt sind aus der Gruppe 30 - 55 Jahre.

4. Verfahren zur Diagnose oder Prognose oder Risikostratifizierung von Patienten mit Testosteronmangel nach einem der vorstehenden Patentansprüche, wobei eine Frühdiagnose oder Früherkennung von Testosteronmangel erfolgt.

5. Verfahren zur Diagnose oder Prognose oder Risikostratifizierung von Patienten mit Testosteronmangel nach einem der vorstehenden Patentansprüche, wobei eine Therapiesteuerung von Testosteronmangel erfolgt, insbesondere Entscheidungen zur Behandlung und Therapie des Patienten, insbesondere Einsatz, Wirkung und / oder Dosierung eines oder mehrerer Arzneimittel, eine therapeutische Maßnahme oder die Überwachung eines Krankheitsverlaufes sowie Therapieverlaufs, Ätiologie oder Klassifizierung einer Erkrankung.

6. Kit zur Durchführung eines Verfahrens nach einem der vorstehenden Patentansprüche, umfassend Materialien zur Analyse des Methylierungsstatus der CpG-Inseln ausgewählt aus mindestens einer Markersequenz MS No. 1-23, insbesondere ein Oligomer davon.
